# EUROPEAN PATENT SPECIFICATION

(11) **EP 3 297 598 B1**
(45) Date of publication and mention of the grant of the patent: **20.03.2019**
(21) Application number: 16713073.1
(22) Date of filing: 15.02.2016
(51) Int. Cl.: A61J 17/00

(54) **PACIFIER**
NUCKEL
TÉTINE

(30) Priority: 19.05.2015 IT UB20150672
(43) Date of publication of application: 28.03.2018
(73) Proprietor: Avanix S.r.l., 24020 Scanzorosciate (Bergamo) (IT)
(72) Inventor: TALLERI, Giuseppe, 24020 Scanzorosciate (Bergamo) (IT); BRIGNOLI, Maurizio, 24020 Scanzorosciate (Bergamo) (IT); MALTESE, Michele, 24020 Scanzorosciate (Bergamo) (IT)
(74) Representative: Gamba, Alessandro
(86) International application number: PCT/IB2016/050797
(87) International publication number: WO 2016/185294

(56) References cited:
- CN-A- 104 257 355
- CN-U- 203 075 245
- US-A1- 2009 024 004
- US-A1- 2009 198 275
- US-A1- 2012 277 794

## Description

The present invention relates to a pacifier for a child or an infant and to a system for monitoring the sleep state of a child and/or a plurality of children comprising, for each child, a respective pacifier.

Preferably, the pacifier object of this invention is suitable to be used by children and infants, and therefore has measures such as to fulfil said purpose.

Pacifiers are used in order to induce sleep or calm a child or infant by stimulating the natural sucking reflex.

The main problem related to the stages of sleep is linked to the fact that during these phases the child is generally not constantly supervised by parents, educators or caregivers. Therefore, the period of sleep is a period of time in which the child is in potential danger. For example, the phenomenon of SIDS, sudden infant death syndrome, happens while the child or infant is sleeping.

In light of this, it is therefore essential to be able to effectively monitor the status of the child or infant while sleeping, allowing parents, educators or caregivers to move away from the crib.

The purpose of this invention is to provide a pacifier suitable to constantly monitor the child or infant and therefore suitable to avoid parents, educators or caregivers from having to constantly stay near the crib. An example of a known pacifier which is suitable to monitor the status of the child or infant is disclosed in document CN203075245. However, as fully evident from the following description, the document CN203075245 is helpful only to defining the general state of the art.

This purpose is achieved by means of the pacifier claimed in claim 1 and by means of the monitoring system claimed in claim 13. The claims dependent on these describe variants of preferred embodiments having further advantageous aspects.

The object of this invention is described below in detail, with the help of the attached tables, wherein:

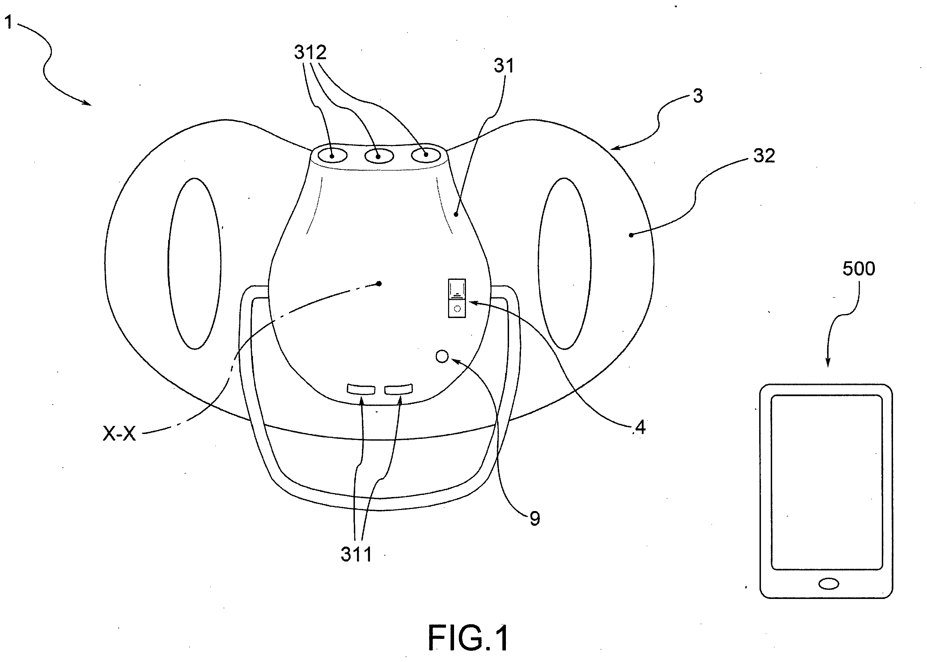

- Figure 1 is a schematic front view of the pacifier object of this invention according to a preferred embodiment and of an electronic device;

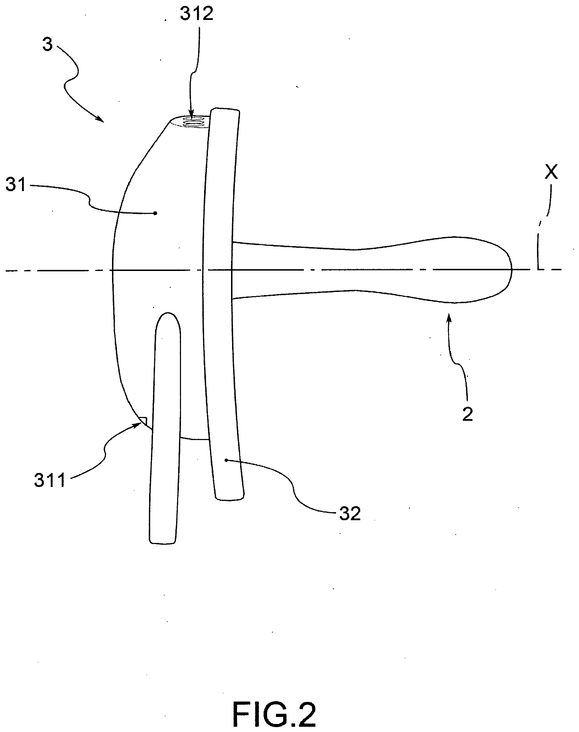

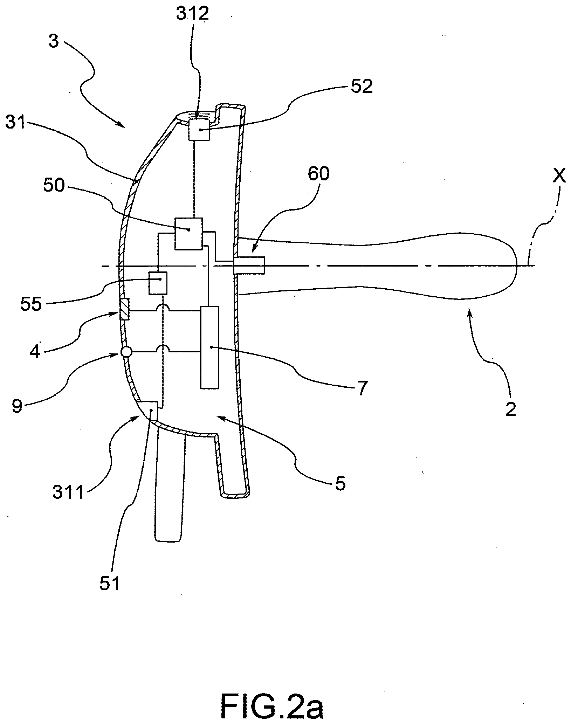

- Figure 2 illustrates a schematic side view of the pacifier object of this invention of Figure 1;
- Figure 2a shows a side view in section of the pacifier of Figure 2;

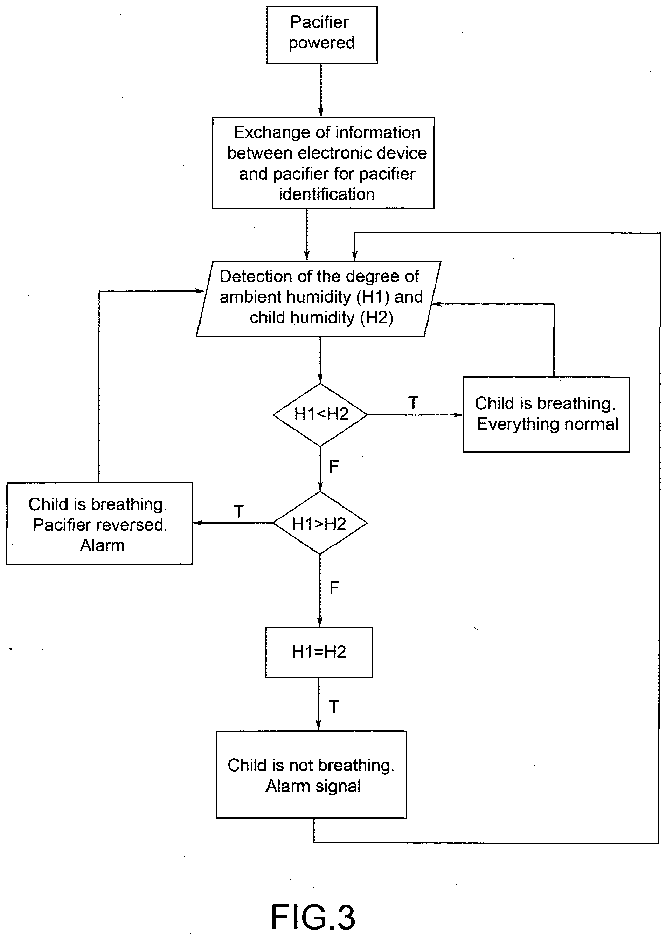

- Figure 3 is a logic block diagram of the operation of the pacifier in a preferred embodiment;

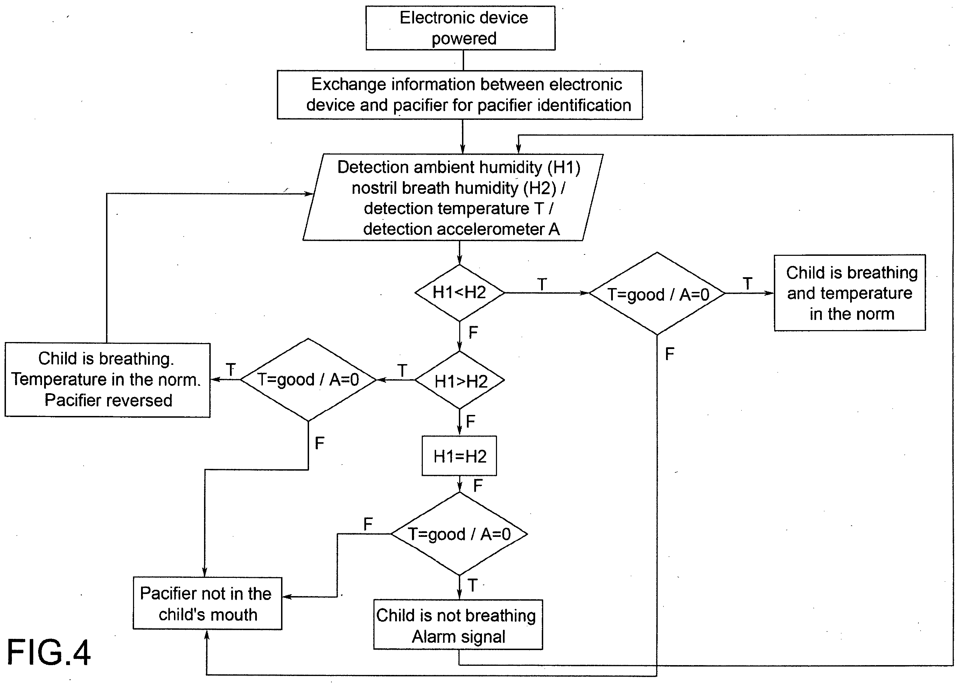

- Figure 4 is a logic block diagram of the operation of the pacifier in a further preferred embodiment.

In the above-mentioned figures, reference number 1 identifies, in its entirety, a pacifier for a child or infant according to the object of this invention.

Preferably, the pacifier 1 is suitable to interact with an electronic device 500.

Preferably, the electronic device 500 is suitable to receive from the pacifier 1 one or more pieces of information and is suited to manage them, for example by storing or combining them with additional information from the device itself. In addition, the electronic device 500 is suitable to allow the use by the user of certain of said information, for example, in visual form, on a screen or by means of a light signal. Additionally, the electronic device 500 is suitable, depending on the information received, to inform a user of it, for example by a sound signal. For example, as subsequently described the electronic device 500 is suitable to emit an alarm signal upon the occurrence of several predefined conditions.

In a preferred embodiment, the electronic device 500 is a mobile phone or a smartphone. In further preferred embodiments, the electronic device 500 is a PC or a tablet.

In yet further other embodiments, the electronic device 500 is a complex system with specific components such as to fulfil the aforesaid functionalities.

Furthermore, the object of this invention is also system for monitoring the state of sleep of a child comprising an electronic device 500 and a pacifier 1 mutually interacting.

In addition, the object of this invention is a system for monitoring the sleep state of a plurality of children comprising an electronic device 500, and a pacifier 1, for each child to be monitored, having the characteristics described below, wherein each pacifier 1 is operatively connected to the electronic device 500.

In particular, in a preferred embodiment, the pacifier 1 comprises a nipple portion 2 suitable to be received in the oral cavity of the child or infant, and a shield portion 3 suitable to abut the lips.

Preferably, both the nipple portion 2 and the shield portion 3 are made of an anti-allergic and sterilisable material.

Preferably, the pacifier 1 has a main axis X-X along which the nipple portion 2 extends. Preferably, the shield portion 3 comprises a central element 31 positioned substantially on the main axis X-X and a shield wall 32 that extends radially from it.

Preferably, the nipple portion 2 is fixed to the central element 31.

Preferably, the shield portion 32 has an anatomical shape to adapt to the child's face.

According to a preferred embodiment, the pacifier 1 also comprises a humidity measurement group 5 suitable to measure variations in humidity surrounding the pacifier 1 when in the child's mouth.

In particular, the humidity measurement group 1 comprises at least an ambient humidity sensor 51 measuring the degree of humidity of the ambient air and creating a corresponding ambient humidity signal H1.

In addition, the humidity measurement group 5 comprises at least a child humidity sensor 52 measuring the degree of humidity of the air emitted from the nostrils of the child and creating a corresponding child humidity signal H2.

In a preferred embodiment, the measurement group 5 is housed in the shield portion 3, preferably in the central element 31.

Preferably, the measurement group 5 is housable in the shield portion 3 in such a way as to be removable from it, for example to clean and sterilise it.

In a preferred embodiment, the child humidity sensor 52 is positioned proximally to the nostrils of the child when the pacifier 1 is housed in the oral cavity.

Preferably, the shield portion 3, in particular the central element 31, is suitably shaped to keep the child humidity sensor 52 in proximity of the nostrils.

In addition, preferably, the ambient humidity sensor 51 is positioned in such a way as not to be influenced by the air exhaled from the child's nostrils.

Preferably, the ambient humidity sensor 51 is positioned distally from the child's nostrils when the pacifier 1 is housed in the oral cavity.

Preferably, the ambient humidity sensor 51 and the child humidity sensor 52 are opposite one another; for example they are radially opposite with respect to the main axis X-X.

In other words, the shield portion 3, preferably its central element 31, has at least one ambient opening 311 and at least one child opening 312 suitable to allow the entry of air into the shield portion 3 up to the respective sensors contained in it.

According to a preferred embodiment, the humidity measurement group 5 comprises a processing and assessment unit 50 operatively connected to said sensors for receiving and comparing the value of the respective signals creating a comparison signal H0.

In other words, the processing and assessment unit 50 fulfils the task of comparing the child humidity signal H2 with the ambient humidity signal H1 identifying a result as the comparison signal H0. In other words, the comparison signal H0 assesses the ratio between the ambient humidity signal H1 over the value of the child humidity signal H2.

In other words, if the signal value H0 is less than 1, it means that the value of the ambient humidity signal H1 is less than the child humidity signal value H2, thus identifying a state of regular sleep, in which the pacifier 1 is in the child's mouth and it is breathing properly its nostrils.

While, if the signal value H0 is equal to, or greater than, 1, it means that the value of the ambient humidity signal H1 is equal to, or greater than, the child humidity signal value H2, thus identifying an abnormal state.

In particular, in fact, ambient humidity signal value H1 is greater than the child humidity signal value H2, this corresponds to the fact that the pacifier 1 is in the child's mouth backwards.

Moreover, in fact, if the ambient humidity signal value H1 is equal to the child humidity signal value H2, this corresponds to the fact that the pacifier 1 is not in the child's mouth, or that the child has stopped breathing, identifying a situation of danger or potential danger.

In particular, if the pacifier is detected, by suitable means (described below), housed in the child's mouth, and the comparison signal H0 is equal to 1, the electronic device 50 is suitable to emit a "child not breathing" alarm signal.

Preferably, the measurement group 5 comprises transmission means 55 suitable to transmit the comparison signal H0 to the electronic device 500. In other words, the transmission means 55 are suitable to transmit the information function of the comparison between the value of the ambient humidity signal H1 and the value of the child humidity signal H2 to the electronic device 500. When the information arrives to the electronic device 500, the latter is suitable to use the information, share it emit an alarm signal.

According to a preferred embodiment, the transmission means 55 and the electronic device 500 are operatively connected by means of wireless transmission, preferably according to Wi-Fi protocol or Bluetooth protocol.

Preferably, the pacifier 1 and the electronic device 500 are suitable to perform a "pairing" procedure for mutual and unequivocal identification.

According to a preferred embodiment, the pacifier 1 also comprises a presence detection group 60 suitable to detect the housing of the pacifier in the oral cavity of the child. Preferably, the detection group 60 is suitable to add a piece of information to the state of sleep of the child by adding a further piece of information with respect to that measured by the humidity measurement group 5.

According to a preferred embodiment, the processing and assessment unit 50 is operatively connected to said presence detection group 60 and is suited to manage the information received; for example the processing and assessment unit 50 is suitable to combine the collected information, to then send it to the electronic device 500 by the transmission means 55.

For example, in a preferred embodiment, the electronic device 50 is suitable to emit a "child not breathing" alarm signal if the presence detection group 60 detects the housing of the pacifier 1 in the oral cavity of the child.

In further other words, the presence detection group 60 is suitable to distinguish a situation of danger from one of potential danger.

This is to say that the presence detection group 60 is suitable to perform a further verification of the state of the child to be added to that, in the first place, performed by the humidity measurement group 5.

According to a preferred embodiment, the presence detection group 60 comprises temperature measurement means comprises temperature detection means suitable for measuring the temperature of the nipple portion 2, and thus suitable to detect if the nipple portion 2 is in the child's mouth, and is thus heated by it, or pacifier 1 is not in the child's mouth and the nipple portion 2 is cooled by the surrounding environment.

Preferably, the temperature measurement means comprise at least one thermocouple. Preferably the thermocouple is accommodated directly in the nipple portion 2.

Preferably, the temperature measurement means comprise at least one infrared temperature sensor. Preferably, the infrared temperature sensor is housable in the shield portion 3.

In a further embodiment, the presence detection group 60 comprises movement detection means suitable to detect the movement of the pacifier 1, and thus suitable to detect if the pacifier 1 is in the child's mouth and moved by its sucking.

Preferably, the movement detection means comprise at least one accelerometer suitable for this purpose.

According to a preferred embodiment, the pacifier 1 also comprises a battery 7, preferably rechargeable, for powering the humidity measurement group 5 and possibly the presence detection group 60.

Preferably, the pacifier 1 comprises a power switch operatively connected to the battery 7, preferably placed on the shield portion 3 on the side opposite to the nipple portion 2.

According to a preferred embodiment, the pacifier 1 also comprises a light emitter 9, for example an LED, positioned on the shield portion 3 and operatively connected to the humidity measurement group 5.

Preferably, the light emitter 9 is suitable to indicate the status of the battery 7, but in further embodiments it is also suitable to indicate a state of danger detected.

Innovatively, the pacifier and the system for monitoring the state of sleep of a child are suitable to fulfil the aforesaid needs and solve the typical problems of the prior art.

Advantageously, in fact, the pacifier is suitable to constantly monitor the child during the entire duration of its sleep.

In addition, advantageously, the pacifier is suitable to effectively monitor the child's condition by constantly checking its proper breathing; the respiratory action of the child or infant cannot, in fact, stop or fail because, if this should occur, a state of danger is identified.

A further advantageous aspect lies in the fact that the pacifier is designed to interact with an electronic device that is suitable to report possible or potential states of danger, thus allowing the parent, educator or caregiver to move away from the crib, taking the electronic device with them.

A still further advantageous aspect is that the pacifier is suitable to interact with communication devices such as mobile phones, smartphones, tablets or PCs. Advantageously, the same electronic devices are suitable to combine a plurality of information contained in them, or detected by them, such as the time, to the information exchanged with the pacifier. Advantageously, the electronic devices are equipped with special applications and software for the management and use of the information.

Advantageously, the pacifier is suitable to distinguish its position in the child's mouth or the situation in which it is no longer in its mouth.

## Claims

1. Pacifier (1) for children or infants suitable to interact with an electronic device (500), preferably a smartphone, comprising:
- a nipple portion (2) suitable to be received in the oral cavity of the child or infant;
- a shield portion (3) suitable to abut the lips of the child or infant;
wherein the pacifier (1) is **characterized by** the fact that it also comprises a humidity measurement group (5) comprising:
i) at least an ambient humidity sensor (51) measuring the degree of humidity of the ambient air and creating a corresponding ambient humidity signal (H1);
ii) at least a child humidity sensor (52) measuring the degree of humidity of the ambient air from the nostrils of the child and creating a corresponding child humidity signal (H2);
iii) a processing and assessment unit (50) operatively connected to said sensors for receiving and comparing the value of the respective signals creating a comparison signal (H0);
iv) transmission means (55) suitable for transmitting the comparison signal (H0) to the electronic device (500).

2. Pacifier (1) according to claim 1, wherein if the comparison signal (H0) is not less than 1, and thus if the value of the ambient humidity signal (H1) is not less than the value of the child humidity signal (H2), the electronic device (50) is suitable to emit an alarm signal.

3. Pacifier (1) according to any of the preceding claims, wherein if the comparison signal (H0) is equal to 1, and thus if the value of the child humidity signal (H2) is equal to the value of the ambient humidity signal (H1), the electronic device (50) is suitable to emit an alarm signal.

4. Pacifier (1) according to any of the previous claims, wherein the measurement group (5) is housed in the shield portion (3).

5. Pacifier (1) according to any of the preceding claims, wherein the child humidity sensor (52) is positioned in the shield portion (3) proximally to the nostrils of the child when the pacifier (1) is housed in the oral cavity.

6. Pacifier (1) according to claim 5, wherein the ambient humidity sensor (51) is positioned in an opposite position with respect to the child humidity sensor (52).

7. Pacifier (1) according to any of the preceding claims, also comprising a presence detection group (60) suitable to detect the housing of the pacifier (1) in the oral cavity of the child, in which the processing and assessment unit (50) is operatively connected to said presence detection group (60) and is suitable for managing the information received.

8. Pacifier (1) according to claim 7 in combination with claim 3, wherein the electronic device (50) is suitable to emit a "child not breathing" alarm signal if the presence detection group (60) detects the housing of the pacifier (1) in the oral cavity of the child.

9. Pacifier (1) according to any of claims 7 and 8, wherein the presence detection group (60) comprises temperature measurement means suitable for measuring the temperature of the nipple portion (2) and/or movement detection means suitable for detecting the sucking movement of the pacifier (1).

10. Pacifier (1) according to any of the preceding claims, wherein the transmission means (55) and the electronic device (500) are operatively connected by means of wireless transmission, preferably according to Wi-Fi protocol or Bluetooth protocol.

11. Pacifier (1) according to any one of the preceding claims, further comprising a battery (7), preferably rechargeable, for powering the humidity measurement group (5) and possibly the presence detection group (60).

12. Pacifier (1) according to claim 10, comprising a power switch operatively connected to the battery (7), preferably placed on the shield portion (3) on the side opposite to the nipple portion (2).

13. Pacifier (1) according to any of the preceding claims, further comprising a light emitter (9), for example an LED, positioned on the shield portion (3), preferably on the side opposite to the nipple portion (2), and operatively connected to the humidity measurement group (5).

14. System for monitoring the sleep state of a child comprising an electronic device (500), and a pacifier (1) according to any of the preceding claims, wherein the pacifier (1) is operatively connected to the electronic device (500).

15. System for monitoring the sleep state of a plurality of children comprising an electronic device (500), and a plurality of pacifiers (1) for each child according to any of the preceding claims, wherein each pacifier (1) is operatively connected to the electronic device (500).

## Patentansprüche

1. Schnuller (1) für Kinder oder Säuglinge, geeignet zur Interaktion mit einer elektronischen Vorrichtung (500), vorzugsweise einem Smartphone, umfassend:
- einen Nippelabschnitt (2), der geeignet ist, in der Mundhöhle des Kindes oder Säuglings aufgenommen zu werden;
- einen Abschirmabschnitt (3), der geeignet ist, um an den Lippen des Kindes oder Säuglings anzuliegen;
wobei der Schnuller (1) **gekennzeichnet ist durch** die Tatsache, dass er auch eine Feuchtigkeitsmessgruppe (5) umfasst, die umfasst:
i) mindestens einen Umgebungsfeuchtesensor (51), der den Feuchtigkeitsgrad der Umgebungsluft misst und ein entsprechendes Umgebungsfeuchtesignal (H1) erzeugt;
ii) mindestens einen Kinderfeuchtesensor (52), der den Feuchtigkeitsgrad der Umgebungsluft aus den Nasenlöchern des Kindes misst und ein entsprechendes Kinderfeuchtesignal (H2) erzeugt;
iii) eine Verarbeitungs- und Bewertungseinheit (50), die operativ mit den Sensoren verbunden ist, um den Wert der jeweiligen Signale zu empfangen und zu vergleichen und ein Vergleichssignal (H0) zu erzeugen;
iv) Übertragungsmittel (55), die zum Übertragen des Vergleichssignals (H0) an die elektronische Vorrichtung (500) geeignet sind.

2. Schnuller (1) nach Anspruch 1, wobei, wenn das Vergleichssignal (H0) nicht kleiner als 1 ist, und somit, wenn der Wert des Umgebungsfeuchtesignals (H1) nicht kleiner als der Wert des Kinderfeuchtesignals (H2) ist, die elektronische Vorrichtung (50) geeignet ist, ein Alarmsignal zu senden.

3. Schnuller (1) nach einem der vorhergehenden Ansprüche, wobei, wenn das Vergleichssignal (H0) gleich 1 ist, und somit, wenn der Wert des Kinderfeuchtesignals (H2) gleich dem Wert des Umgebungsfeuchtesignals (H1) ist, die elektronische Vorrichtung (50) geeignet ist, ein Alarmsignal auszusenden.

4. Schnuller (1) nach einem der vorhergehenden Ansprüche, wobei die Messgruppe (5) im Abschirmabschnitt (3) untergebracht ist.

5. Schnuller (1) nach einem der vorhergehenden Ansprüche, wobei der Kinderfeuchtesensor (52) im Schildabschnitt (3) nahe den Nasenlöchern des Kindes positioniert ist, wenn der Schnuller (1) in der Mundhöhle untergebracht ist.

6. Schnuller (1) nach Anspruch 5, wobei der Umgebungsfeuchtesensor (51) in einer entgegengesetzten Position in Bezug auf den Kinderfeuchtesensor (52) positioniert ist.

7. Schnuller (1) nach einem der vorhergehenden Ansprüche, auch umfassend eine Anwesenheitserkennungsgruppe (60), die geeignet ist, das Gehäuse des Schnullers (1) in der Mundhöhle des Kindes zu erfassen, wobei die Verarbeitungs- und Beurteilungseinheit (50) operativ mit der Anwesenheitserkennungsgruppe (60) verbunden ist und zum Verwalten der empfangenen Informationen geeignet ist.

8. Schnuller (1) nach Anspruch 7 in Kombination mit Anspruch 3, wobei die elektronische Vorrichtung (50) geeignet ist, ein Alarmsignal "Kind atmet nicht" auszusenden, wenn die Anwesenheitserfassungsgruppe (60) das Gehäuse des Schnullers (1) in der Mundhöhle des Kindes erfasst.

9. Schnuller nach einem der Ansprüche 7 und 8, wobei die Anwesenheitserfassungsgruppe (60) Temperaturmessmittel umfasst, die zum Messen der Temperatur des Nippelabschnitts (2) und/oder Bewegungserfassungsmittel, die zum Erfassen der Saugbewegung des Schnullers (1) geeignet sind.

10. Schnuller (1) nach einem der vorstehenden Ansprüche, wobei die Übertragungsmittel (55) und die elektronische Vorrichtung (500) mittels drahtloser Übertragung, vorzugsweise nach dem Wi-Fi-Protokoll oder Bluetooth-Protokoll, operativ verbunden sind.

11. Schnuller (1) nach einem der vorstehenden Ansprüche, ferner umfassend eine Batterie (7), vorzugsweise wiederaufladbar, zum Betreiben der Feuchtemessgruppe (5) und gegebenenfalls der Anwesenheitserfassungsgruppe (60).

12. Schnuller (1) nach Anspruch 10, umfassend einen Stromschalter, der funktionsfähig mit der Batterie (7) verbunden ist, vorzugsweise auf dem Abschirmabschnitt (3) auf der dem Nippelabschnitt (2) gegenüberliegenden Seite angeordnet.

13. Schnuller (1) nach einem der vorstehenden Ansprüche, ferner umfassend einen Lichtemitter (9), zum Beispiel eine LED, der auf dem Abschirmabschnitt (3), vorzugsweise auf der dem Nippelabschnitt (2) abgewandten Seite, angeordnet und operativ mit der Feuchtemessgruppe (5) verbunden ist.

14. System zum Überwachen des Schlafzustands eines Kindes, umfassend eine elektronische Vorrichtung (500) und einen Schnuller (1) nach einem der vorhergehenden Ansprüche, wobei der Schnuller (1) in der elektronischen Vorrichtung (500) operativ verbunden ist.

15. System zum Überwachen des Schlafzustands einer Vielzahl von Kindern, umfassend eine elektronische Vorrichtung (500) und eine Vielzahl von Schnullern (1) für jedes Kind gemäß einem der vorhergehenden Ansprüche, wobei jeder Schnuller (1) operativ mit der elektronischen Vorrichtung (500) verbunden ist.

## Revendications

1. Tétine (1) pour des enfants ou des nouveau-nés adaptée pour interagir avec un dispositif électronique (500), de préférence un smartphone, comprenant :
- une portion de tétine (2) adaptée pour être reçue dans la cavité orale de l'enfant ou du nouveau-né ;
- une portion de protection (3) adaptée pour buter contre les lèvres de l'enfant ou du nouveau-né ;
dans laquelle la tétine (1) est **caractérisée par le fait qu'**elle comprend aussi un groupe de mesure d'humidité (5) comprenant :
i) au moins un capteur d'humidité ambiant (51) mesurant le degré d'humidité de l'air ambiant et créant un signal d'humidité ambiante correspondant (H1) ;
ii) au moins un capteur d'humidité d'enfant (52) mesurant le degré d'humidité de l'air ambiant provenant des narines de l'enfant et créant un signal d'humidité d'enfant correspondant (H2) ;
iii) une unité de traitement et d'évaluation (50) raccordée en fonctionnement auxdits capteurs pour recevoir et comparer la valeur des signaux respectifs créant un signal de comparaison (H0) ;
iv) un moyen de transmission (55) adapté pour transmettre le signal de comparaison (H0) au dispositif électronique (500).

2. Tétine (1) selon la revendication 1, dans laquelle si le signal de comparaison (H0) n'est pas inférieur à 1, et ainsi si la valeur du signal d'humidité ambiante (H1) n'est pas inférieure à la valeur du signal d'humidité d'enfant (H2), le dispositif électronique (50) est adapté pour émettre un signal d'alarme.

3. Tétine (1) selon l'une quelconque des revendications précédentes, dans laquelle si le signal de comparaison (H0) est égal à 1, et ainsi si la valeur du signal d'humidité d'enfant (H2) est égale à la valeur du signal d'humidité ambiante (H1), le dispositif électronique (50) est adapté pour émettre un signal d'alarme.

4. Tétine (1) selon l'une quelconque des revendications précédentes, dans laquelle le groupe de mesure (5) est logé dans la portion de protection (3).

5. Tétine (1) selon l'une quelconque des revendications précédentes, dans laquelle le capteur d'humidité d'enfant (52) est positionné dans la portion de protection (3) à proximité des narines de l'enfant lorsque la tétine (1) est logée dans la cavité orale.

6. Tétine (1) selon la revendication 5, dans laquelle le capteur d'humidité ambiante (51) est positionné dans une position opposée par rapport au capteur d'humidité d'enfant (52).

7. Tétine (1) selon l'une quelconque des revendications précédentes, comprenant ainsi un groupe de détection de présence (60) adapté pour détecter le logement de la tétine (1) dans la cavité orale de l'enfant, dans lequel l'unité d'évaluation (50) est raccordée en fonctionnement audit groupe de détection de présence (60) et est adaptée pour gérer l'information reçue.

8. Tétine (1) selon la revendication 7 en combinaison avec la revendication 3, dans laquelle le dispositif électronique (50) est adapté pour émettre un signal d'alarme « enfant ne respirant pas » si le groupe de détection de présence (60) détecte le logement de la tétine (1) dans la cavité orale de l'enfant.

9. Tétine (1) selon l'une quelconque des revendications 7 et 8, dans laquelle le groupe de détection de présence (60) comprend un moyen de mesure de température adapté pour mesurer la température de la portion de tétine (2) et/ou un moyen de détection de mouvement adapté pour détecter le mouvement de succion de la tétine (1).

10. Tétine (1) selon l'une quelconque des revendications précédentes, dans laquelle le moyen de transmission (55) et le dispositif électronique (500) sont raccordés en fonctionnement à l'aide de la transmission sans fil, de préférence selon un protocole Wi-Fi ou protocole Bluetooth.

11. Tétine (1) selon l'une quelconque des revendications précédentes, comprenant en outre une batterie (7), de préférence rechargeable, pour alimenter le groupe de mesure d'humidité (5) et éventuellement le groupe de détection de présence (60).

12. Tétine (1) selon la revendication 10, comprenant un commutateur d'alimentation raccordé en fonctionnement à la batterie (7), placé de préférence sur la portion de protection (3) sur le côté opposé à la portion de tétine (2).

13. Tétine (1) selon l'une quelconque des revendications précédentes, comprenant en outre un émetteur de lumière (9), par exemple une DEL, positionné sur la portion de protection (3), de préférence sur le côté opposé à la portion de tétine (2), et raccordé en fonctionnement au groupe de mesure d'humidité (5).

14. Système de surveillance de l'état de sommeil d'un enfant comprenant un dispositif électronique (500), et une tétine (1) selon l'une quelconque des revendications précédentes, dans laquelle la tétine (1) est raccordée en fonctionnement au dispositif électronique (500).

15. Système de surveillance de l'état de sommeil d'une pluralité d'enfants comprenant un dispositif électronique (500), et une pluralité de tétines (1) pour chaque enfant selon l'une quelconque des revendications précédentes, dans lequel chaque tétine (1) est raccordée en fonctionnement au dispositif électronique (500).
